# EUROPEAN PATENT APPLICATION

(11) **EP 4 640 272 A1**
(43) Date of publication of application: **29.10.2025**
(21) Application number: 25161358.4
(22) Date of filing: 03.03.2025
(51) Int. Cl.: A61N 7/02, A61B 18/00, A61N 7/00

(54) **HIGH INTENSIVE FOCUSED ULTRASOUND PROBE**

(30) Priority: 23.04.2024 KR 20240053766
(71) Applicant: VIOL CO., LTD., Seongnam-si, Gyeonggi-do 13510 (KR)
(72) Inventor: LEE, Sangjin, Seongnam-si 13510 (KR); JEON, Yeonggi, Seongnam-si 13510 (KR)
(74) Representative: Zimmermann, Tankred Klaus

(57) **Abstract**

Disclosed is a high-intensity focused ultrasound (HIFU) probe for noninvasive skin treatment. The probe includes a cartridge with an internal space filled with a liquid ultrasound transmission medium, a handpiece to which the cartridge is coupled, and a transducer within the cartridge that generates and emits HIFU. A shaft guides the transducer's linear motion in a specific direction and is formed as a hollow pipe. This hollow shaft functions as a heat exchange pipe, circulating a cooling medium to regulate the temperature of the liquid ultrasound transmission medium, preventing overheating during operation.

## Description

### [Technical Field]

Embodiments of the present disclosure relate to an ultrasound probe, and particularly, to a high intensive focused ultrasound probe that noninvasively treats skin by using a high intensive focused ultrasound (HIFU).

### [Background Art]

Skin procedure using a high intensive focused ultrasound (HIFU) has recently been in spotlight. This is a technology of treating skin by using an effect (wrinkle removal, skin elasticity improvement, and the like) occurring when high intensive acoustic energy is focused on a local site in a body by using the high intensive focused ultrasound to increase temperature and thus a degenerated tissue is regenerated due to thermal variations occurring in the local site in the body.

A device that treats skin by using the high intensive focused ultrasound includes a transducer. The transducer generates high intensive ultrasound from an input power source and outputs the high intensive ultrasound. A general high intensive focused ultrasound device uses a circular single-element ultrasound transducer as a transducer. That is, a method is used to transmit strong ultrasound energy to a treatment site through the circular single-element ultrasound transducer.

A high intensive focused ultrasound device in the related art generally includes the transducer in a cartridge portion that physically comes into direct contact with the skin. In order to more effectively transmit high intensive focused ultrasound generated by the transducer to the skin, the inside of the cartridge is filled with a liquid ultrasound transmission medium, and degassed water (water from which air has been removed) is generally used as the ultrasound transmission medium.

As described above, the ultrasound transmission medium vibrates due to ultrasound and transmits the high intensive focused ultrasound generated by the transducer to the skin. Therefore, in the process of transmitting the ultrasound, temperature inevitably increases (increases up to about 45°C to 50°C) due to friction between particles caused by the vibration. As a result, there is a problem in that a user may feel discomfort and may be burned in severe cases.
Korean Patent Publication No. 10-2012-0140288 (Published on December 31, 2012)
Korean Patent Publication No. 10-2014-0141062 (Published on December 10, 2014)

### [Disclosure]

### [Technical Problem]

An object of the present disclosure is to provide a high intensive focused ultrasound probe that can effectively suppress an increase in the temperature of an ultrasound transmission medium (for example, degassed water) filled in the high intensive focused ultrasound probe.

Problems to be solved by the present disclosure are not limited to the aforementioned problems, and the other unmentioned problems will be clearly understood by those skilled in the art from the following description.

### [Technical Solution]

The present disclosure provides a high intensive focused ultrasound probe that emits high intensive focused ultrasound to skin, the high intensive focused ultrasound probe including a cartridge having an internal space filled with a liquid ultrasound transmission medium, a handpiece to which the cartridge is coupled, a transducer arranged in the internal space of the cartridge and configured to generate high intensive focused ultrasound from an input power source and output the generated high intensive focused ultrasound, and a shaft configured to guide a one-dimensional linear motion of the transducer with respect to a specific direction in the internal space

In an embodiment, the shaft may be configured in a form of a hollow pipe having an empty interior, and the shaft having the form of a hollow pipe may serve as a heat exchange pipe that circulates a cooling medium in the internal space. In this case, an increase in the temperature of a liquid ultrasonic transmission medium filled in the internal space can be suppressed by the cooling effect of the cooling medium circulating in the internal space along the shaft.

In an embodiment, the shaft may include a first pipe portion into which the cooling medium is introduced through an inlet formed on a side of a first sidewall of the cartridge, a second pipe portion parallel to the first pipe portion and through which the cooling medium is discharged through an outlet formed on the side of the first sidewall, and a connection pipe portion configured to connect the first pipe portion and the second pipe portion so that the cooling medium is able to flow.

As an embodiment, at least a part of the connection pipe portion may protrude outward from a second sidewall of the cartridge on an opposite side of the first sidewall and may be exposed to the outside (air). In this case, some of heat is released into the air while the cooling medium passes through the connection pipe portion exposed to the outside, so that the cooling medium can recover its cooling performance.

Heat dissipation fins may also be attached to a surface of the connection pipe portion protruding outward from the first sidewall and exposed to the outside. In this case, the cooling performance of the cooling medium can be significantly recovered by the heat dissipation fins attached to the surface of the connection pipe portion.

As another embodiment, the connection pipe portion may also be arranged in the internal space of the cartridge. In this case, since the connection pipe portion is not exposed to the outside, it is difficult to expect an effect of recovering the cooling performance of the cooling medium through heat exchange with the outside air, but the structure is advantageous in terms of miniaturization or compactness of the device.

In an embodiment, a supply pipe introduced from an outside into an inside of the handpiece may be connected to the inlet of the first pipe portion through a first connection port, and a discharge pipe drawn out from the inside of the handpiece to the outside may be connected to the outlet of the second pipe portion through a second connection port.

In an embodiment, the cooling medium may be water or air.

When the cooling medium is water, a check valve, which prevents the backflow of the cooling medium, that is, allows cooling water to flow only in one predetermined direction, can be installed in the first connection port and the second connection port.

In this case, the check valve of the first connection port can be arranged so that the cooling medium flows only in the direction from the supply pipe to the first pipe portion, and the check valve of the second connection port can be arranged so that the cooling medium flows only in the direction from the second pipe portion to the discharge pipe.

In an embodiment, the transducer may be connected to a movable block moving along the shaft and may make a one-dimensional linear reciprocating motion in a specific direction together with the movable block.

A high intensive focused ultrasound probe according to the present disclosure may further include an external rotating shaft configured to rotate inside the handpiece by a motor, an internal rotating shaft configured to move the transducer while rotating inside the cartridge, and a magnet coupler configured to magnetically couple the external rotating shaft and the internal rotating shaft with a first sidewall of the cartridge interposed between the external rotating shaft and the internal rotating shaft.

The magnet coupler may include a first coupler coupled to the external rotating shaft and making a synchronized rotational motion, and a second coupler coupled to the internal rotating shaft and forming magnetic coupling with the first coupler with the first sidewall interposed between the first coupler and the second coupler

As an embodiment, one of the first coupler and the second coupler may be a magnetic material and the other one of the first coupler and the second coupler may be a permanent magnet.

As another embodiment, the first coupler and the second coupler may be permanent magnets, and a magnetic pole of a surface of the first coupler and a magnetic pole of a surface of the second coupler may be opposite to each other, the two surfaces being in close contact with each other with the first sidewall interposed therebetween.

As another embodiment, a plurality of coupling magnets may be separately mounted at uniform intervals along a rotation direction on a surface of the first coupler and a surface of the second coupler, the two surfaces facing each other with the first sidewall interposed therebetween. In this case, coupling magnets mounted on the surface of the first coupler may be arranged so that magnetic poles of sides exposed to an outside are alternated with respect to the rotation direction, and coupling magnets mounted on the surface of the second coupler may be arranged so that magnetic poles of sides exposed to the outside are alternated with respect to the rotation direction.

A plurality of balls or needle pins may be installed on the surface of the first coupler and the surface of the second coupler, the two surfaces facing each other with the first sidewall interposed therebetween. In this case, at least a part of the balls or the needle pins may protrude from the surfaces facing each other to support rotational motions of the first coupler and the second coupler in a state of being in contact with the first sidewall.

A ring-shaped internal rotation guide may be further formed on an inner surface of the first sidewall where the magnet coupler is located. In addition, a ring-shaped external rotation guide may be further formed on an outer surface of the first sidewall corresponding to the internal rotation guide.

In this case, the magnet coupler may be arranged in an internal coupler receiving portion and an external coupler receiving portion respectively partitioned in an inside and an outside of the first sidewall by the internal rotation guide and the external rotation guide

A first lubricating layer may be formed by a lubricant between the first coupler and a first sidewall of the external coupler receiving portion. In addition, a second lubricating layer may be formed by a lubricant between the second coupler and a first sidewall of the internal coupler receiving portion.

In an embodiment, the internal rotating shaft may be configured in the form of a lead screw having threads formed along its peripheral surface, and the movable block may be formed with a fastening hole screw-coupled with the threads of the internal rotating shaft. Accordingly, when the internal rotating shaft rotates, the movable block moves linearly along the internal rotating shaft according to the rotational motion, so that skin treatment or handling can be performed on a wider site.

In an embodiment, the internal space of the cartridge may be divided by a space partition plate into a first space and a second space isolated from the first space.

In this case, the first space may be filled with a liquid ultrasound transmission medium, and the transducer and the internal rotating shaft may be arranged in the first space filled with the liquid ultrasound transmission medium.

A circuit board that controls the transducer may be arranged in the second space.

In an embodiment, at least two detection elements that detect a position of the movable block may be mounted at a distance from each other on the circuit board. In addition, an element to be detected may be arranged on a surface of the movable block adjacent to the circuit board.

Preferably, the detection element may be a Hall element, and the element to be detected may be a permanent magnet.

### [Advantageous Effects]

According to the present disclosure, the high intensive focused ultrasound probe is configured so that the cooling medium (cooling water or cold air) moves along the shaft that guides the linear motion of the transducer within the cartridge and cools the liquid ultrasound transmission medium (for example, degassed water) filled in the cartridge. Accordingly, an increase in the temperature of the cartridge can be suppressed, and problems of the related art, such as discomfort or burns caused by an increase in the temperature of the cartridge, can be solved.

In particular, since the shaft also serves as the heat exchange pipe (cooling pipe) for cooling the ultrasound transmission medium, no separate additional configuration is required to suppress an increase in the temperature of the cartridge. That is, it has the advantage of being an efficient configuration that can implement stable linear motion of the transducer and cartridge cooling with one shaft, and since no separate additional configuration is required for cooling implementation, a high-functionality product with an added cooling function can be provided at low cost.

### [Description of Drawings]

FIG. 1 is a device configuration diagram schematically illustrating the overall configuration of a skin treatment device adopting a high intensive focused ultrasound probe according to embodiments of the present disclosure.
FIG. 2 is a schematic view schematically illustrating a main configuration of the high intensive focused ultrasound probe according to embodiments of the present disclosure.
FIG. 3 is a planar schematic view illustrating a preferred embodiment of a shaft arranged in an internal space (first space) of a cartridge of the high intensive focused ultrasound probe according to embodiments of the present disclosure.
FIG. 4 is a planar schematic view of the present disclosure illustrating a preferred another embodiment of a shaft.
FIG. 5 is a view illustrating a preferred embodiment of a magnet coupler illustrated in FIG. 2.
FIG. 6 is a view illustrating another preferred embodiment of the magnet coupler illustrated in FIG. 2.
FIG. 7 is a view illustrating further another preferred embodiment of the magnet coupler illustrated in FIG. 2.
FIG. 8 is a view illustrating a preferred modified example of the magnet coupler illustrated in FIG. 2.
FIG. 9 is a view illustrating a preferred modified example of a first sidewall illustrated in FIG. 2.

### [Mode for Invention]

Hereinafter, preferred embodiments of the present disclosure are described in detail with reference to the accompanying drawings.

For reference, in the description of embodiments of the present disclosure, the same or similar components are given the same reference numerals and duplicate descriptions thereof are omitted. Even when it is determined that detailed descriptions of related publicly-known technologies may obscure the subject matter of the embodiments disclosed in the present specification, the detailed descriptions thereof are omitted.

In addition, the suffixes "module" and "unit" for components used in the following description are given or used interchangeably only for the convenience of writing a specification, and do not have distinct meanings or roles in themselves.

In addition, in the description of embodiments of the present disclosure, it should be noted that the accompanying drawings are intended only to help easily understand the embodiments disclosed in the present specification and are not intended to limit the technical spirit disclosed in the present specification, and the present disclosure includes all modifications, equivalents, or substitutes included in the spirit and technical scope of the present disclosure.

In addition, in the description of embodiments of the present disclosure, terms including ordinal numbers such as first and second may be used to describe various components, but the components are not limited by the terms. The terms are used only to distinguish one component from another component.

In addition, when it is described that one component is "connected" or "coupled" to another component, it should be understood that one component may be directly connected or coupled to the another component, but another component may exist between the two components.

On the other hand, when it is described that one component is "directly connected to" or "directly coupled to" another component, it should be understood that another component does not exist between the two components.

In addition, terms such as "includes", "comprises" or "has" used in the description of embodiments of the present disclosure are intended to designate the presence of features, numbers, steps, operations, components, parts, or combinations of the present disclosure, and should be understood as not excluding in advance the possibility of the presence or addition of one or more other features, numbers, steps, operations, components, parts or combinations thereof.

In addition, the fact that a component is "in front," "behind," "above," or "below" another component includes not only the case where it is directly adjacent to the another component and is disposed "in front," "behind," "above," or "below," but also the case where another component is further disposed therebetween unless otherwise specified.

The drawings are intended only to help understand the spirit of the present disclosure, and should not be construed as limiting the scope of the present disclosure. In addition, it should be noted that the relative thickness, length, or size in the drawings may be exaggerated for the convenience and clarity of explanation.

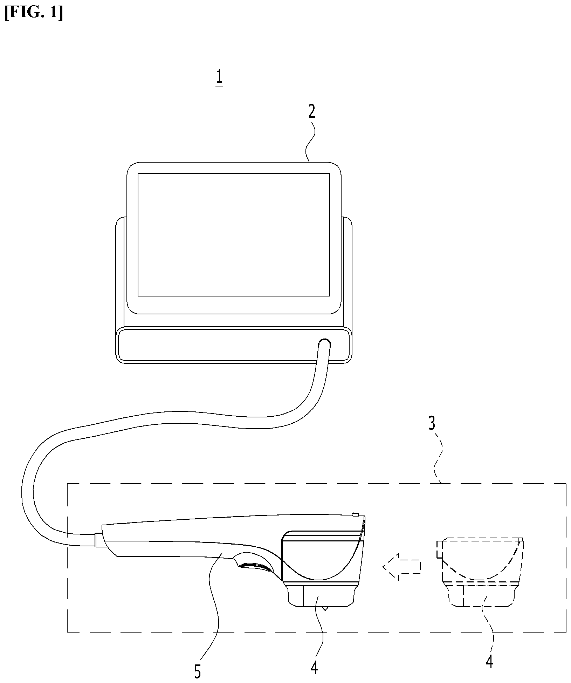

FIG. 1 is a device configuration diagram schematically illustrating the overall configuration of a skin treatment device adopting a high intensive focused ultrasound probe according to embodiments of the present disclosure. First, the configuration of the skin treatment device adopting the high intensive focused ultrasound probe according to embodiments of the present disclosure is briefly described with reference to FIG. 1.

The skin treatment device related to the present disclosure is a device that noninvasively treats or handles skin by using an effect (wrinkle removal, subcutaneous fat removal, skin elasticity improvement, and the like) occurring when high intensive acoustic energy is focused on a local site in a body by using high intensive focused ultrasound to increase temperature and thus a degenerated tissue is regenerated due to thermal variations occurring in the local site in the body.

Referring to FIG. 1, a skin treatment device 1 includes a main body 2 and a high intensive focused ultrasound probe 3 (hereinafter, referred to as an 'ultrasound probe' for convenience of explanation). The main body 2 controls the ultrasound probe 3. High intensive focused ultrasound is generated from the ultrasound probe 3 under the control of the main body 2, and the generated high intensive focused ultrasound can induce thermal variations by being focused on the inside of the body (for example, a dermis layer) through the ultrasound probe 3.

The main body 2 may include an input unit (not illustrated) for user input. The input unit may include not only a mouse and a keyboard but also a mechanical or electronic user interface (for example, a touch input-capable display) implemented in the device. Of course, the present disclosure is not limited thereto, and the input unit is applicable without particular limitations in the method and form thereof as long as the input unit is capable of inputting a user command.

The main body 2 may include an output unit (reference numeral is omitted) that displays information to the outside and transmits the information to a user. The output unit may include, for example, a display, an LED, a speaker, and the like for displaying visual output, auditory output, or tactile output. When the ultrasound probe 3 includes an imaging transducer element module, the output unit may display an ultrasound image of an internal tissue of the body.

The main body 2 may further include a peripheral device interface for data transmission with various types of external devices. For example, the main body 2 may include a memory card port, an external device input/output (I/O) port, and the like. The main body 2 can be connected to the ultrasound probe 3 through wired or wireless communication means to control the ultrasound probe 3.

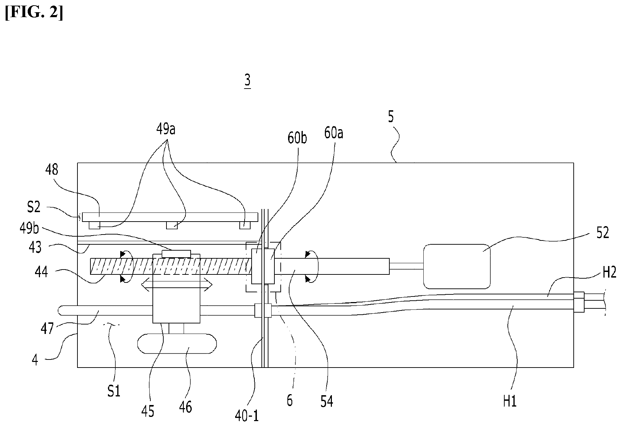

The ultrasound probe 3 may include a cartridge 4 and a handpiece 5. The cartridge 4 may be provided therein with a transducer 46 (see FIG. 2) that generates high intensive focused ultrasound. The cartridge 4 provided with the transducer 46 can be detachably coupled to the handpiece 5 to be replaced with a cartridge 4 that generates high intensive focused ultrasound suitable for a treatment purpose or a medical procedure site and can be used.

The transducer 46 can generate high intensive focused ultrasound from an input power source and output the high intensive focused ultrasound. The cartridge 4 provided with the transducer 46 can be electrically connected or coupled to the handpiece 5 side, and the handpiece 5 can be connected to the main body by wire or wirelessly as described above, thereby exchanging signals or information (for example, transducer driving or control signals and transducer position information).

The cartridge 4 and the handpiece 5 can be electrically and physically connected to each other. The cartridge 4 and the handpiece 5 can be electrically connected to each other by connecting connection terminals (reference numerals are omitted) that are provided to correspond to each other when coupled, and can be physically coupled to each other through a predetermined coupling structure. The coupling structure may include, for example, a bar or a protrusion protruding in a direction in which the cartridge 4 is coupled to a front end of the handpiece 5.

With reference to FIG. 2, the configuration of the high intensive focused ultrasound probe according to embodiments of the present disclosure is described.

FIG. 2 is a schematic configuration view of the high intensive focused ultrasound probe according to embodiments of the present disclosure.

Referring to FIG. 2, the ultrasound probe 3 includes the cartridge 4 and the handpiece 5. The transducer 46 is provided inside the cartridge 4, and the cartridge 4 provided therein with the transducer 46 can be detachably coupled to the handpiece 5. Accordingly, the cartridge 4 can be replaced with a new one, or replaced with an appropriate cartridge 4 according to a treatment purpose or a medical procedure site.

The transducer 46 can generate high intensive focused ultrasound from an input power source and output the high intensive focused ultrasound. As described above, the cartridge 4 provided therein with the transducer 46 can be electrically connected or coupled to the handpiece 5 side. The handpiece 5 can be connected to the main body 2 (see FIG. 1) by wire or wirelessly, thereby exchanging signals or information (for example, transducer driving or control signals and transducer position information).

The cartridge 4 and the handpiece 5 can be electrically connected to each other by connecting electrical connection terminals (not illustrated) that are provided to correspond to each other when coupled. The cartridge 4 and the handpiece 5 can also be physically coupled to each other through a predetermined coupling structure. The coupling structure may include, for example, a bar or a protrusion (not illustrated) protruding in the direction in which the cartridge 4 is coupled to the front end of the handpiece 5.

According to an embodiment, the ultrasound probe 3 and the handpiece 5 may also be configured as an integrated type other than a detachable type as illustrated in the drawing (FIG. 2). In this case, the coupling structure (coupling structure such as a bar or a protrusion that protrudes in the direction in which the cartridge is coupled to the front end of the handpiece as a structure for physically coupling the cartridge and the handpiece) as in the detachable type may be deleted.

The inside of the cartridge 4 may be filled with a liquid ultrasound transmission medium (reference numeral is omitted). The liquid ultrasound transmission medium may be degassed water (water from which air bubbles have been removed). The transducer 46 can be arranged inside the cartridge 4 filled with the liquid ultrasound transmission medium. The transducer 46 can be provided to enable a one-dimensional motion, that is, a linear motion, inside the cartridge 4 in a specific direction (left and right direction based on FIG. 2).

A motor 52 can be installed in the handpiece 5 to implement a one-dimensional motion (linear motion) of the ultrasound transducer 46 in the specific direction. The transducer 46 can be mechanically connected to the motor 52 to receive power from the motor 52, and can generate high intensive focused ultrasound by making a linear reciprocating motion in the specific direction within the cartridge 4 by the received power, thereby focusing the generated high intensive focused ultrasound inside the body.

A space partition plate 43 can be installed in the cartridge 4. An internal space of the cartridge 4 can be divided into a first space S1 and a second space S2 that are isolated from each other by the space partition plate 43. In the first space S1, the transducer 46 can be arranged and the liquid ultrasound transmission medium can be filled, and in the second space S2, a circuit board 48 that controls the operation of the transducer 46 from an input signal can be arranged.

In the first space S1, an internal rotating shaft 44 can be arranged together with the transducer 46. The internal rotating shaft 44 can be physically coupled to an external rotating shaft 54 arranged inside the handpiece 5 to receive rotational force from the external rotating shaft 54, and the transducer 46 can focus high intensive focused ultrasound inside the body while making a linear reciprocating motion in the first space S1 in the specific direction according to the rotation of the internal rotating shaft 44 by the external rotating shaft 54.

The external rotating shaft 54 can be directly connected to an output shaft (reference numeral is omitted) of the motor 52 inside the handpiece 5. The motor 52 can be arranged inside the handpiece 5 together with the external rotating shaft 54. The motor 52 can output rotational force in the forward/reverse direction according to a driving signal of the main body described above. Accordingly, the external rotating shaft 54 rotates in the forward or reverse direction within a set range, and as a result, the internal rotating shaft 44 can also be rotated in the same direction.

The internal rotating shaft 44 can be in the form of a lead screw having threads formed along its peripheral surface. The transducer 46 can be connected to the internal rotating shaft 44 in the first space S1 through a movable block 45, and the movable block 45 can be screw-coupled with the internal rotating shaft 44 through a fastening hole. Accordingly, the transducer 46 can make a linear motion together with the movable block 45 moving on the internal rotating shaft 44 when the internal rotating shaft 44 rotates.

A shaft 47 can be installed in the first space S1. The shaft 47 guides the one-dimensional linear motion of the transducer (46), more specifically, the movable block 45 supporting the transducer (46) in the first space S1 with respect to the specific direction. Thus, the one-dimensional linear motion (linear reciprocating motion according to the rotation of the internal rotating shaft) of the movable block 45 in the first space S1 can be stably implemented without shaking.

The shaft 47 can serve as a thermal exchange pipe that circulates a cooling medium in the first space S1. To this end, the shaft 47 can be configured in the form of a hollow pipe with an empty interior. In this case, the cooling medium can take away heat from the liquid ultrasound transmission medium while moving along the shaft 47 in the form of a hollow pipe (along the internal space of the shaft). That is, overheating of the ultrasound transmission medium can be suppressed by the cooling effect of the cooling medium flowing through the shaft 47.

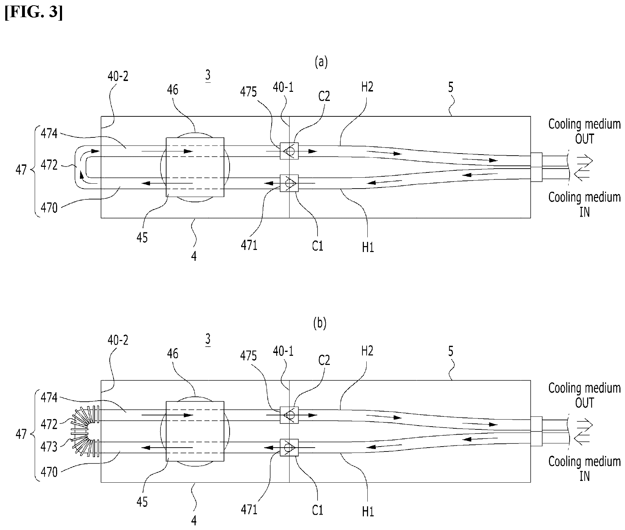

The shaft 47, which serves to guide the movable block 45 supporting the transducer 46 within the cartridge 4 to be able to stably make a linear motion and to cool the liquid ultrasound transmission medium in the inside (first space S1) of the cartridge 4, is described below in more detail with reference to FIG. 3.

A detection element 49a can be mounted on the circuit board 48 arranged in the second space S2. An element 49b to be detected can be provided on the movable block 45 arranged to be movable in the first space S1. The detection element 49a can recognize the element 49b to be detected and generate a corresponding signal to provide the generated signal to the circuit board 48, and a control circuit of the circuit board 48 can process the signal of the detection element 49a to calculate the value of a current position (position in the above specific direction) of the movable block 45.

The detection element 49a can be mounted on one surface of the circuit board 48, more specifically, on the surface of the circuit board 48 facing the movable block 45. At least two detection elements 49a can be mounted at a distance from each other on the one surface of the circuit board 48 along a direction parallel to the movement direction of the movable block 45, and the element 49b to be detected can be attached and fixed to the surface of the movable block 45 adj acent to the circuit board 48 or facing the circuit board 48.

The detection element 49a may be a Hall sensor. The element 49b to be detected may be a permanent magnet. In this case, the Hall sensor can detect the position of the permanent magnet on the movable block 45 by using the Hall effect and generate a corresponding signal to output the generated signal to the circuit board 48, and the circuit board 48 can recognize the position of the movable block 45 from the signal of the Hall sensor. The recognized position value can be utilized to control the position of the movable block 45 by the motor 52.

FIG. 3 is a planar schematic view illustrating a preferred embodiment of the shaft arranged in the internal space (first space) of the cartridge of the high intensive focused ultrasound probe according to embodiments of the present disclosure.

Referring to FIG. 3 and the preceding FIG. 2, the shaft 47 serves as a guide that guides the linear motion of the movable block 45 and a heat exchanger pipe. In an embodiment, the shaft 47 may include a first pipe portion 470 parallel to the internal rotating shaft 44 and a second pipe portion 474 parallel to the first pipe portion 470. The shaft 47 may also have a connection pipe portion 472 connecting the first pipe portion 470 and the second pipe portion 474.

The first pipe portion 470 may have an inlet 471 on the first sidewall 40-1 side of the cartridge 4. The second pipe portion 474 may have an outlet 475 on the first sidewall 40-1 side. The cooling medium is introduced into the first pipe portion 470 through the inlet 471, sequentially passes through the connection pipe portion 472 and the second pipe portion 474, and then exits the cartridge 4 through the outlet 475. In this process, the ultrasound transmission medium can be cooled by the cooling effect of the cooling medium.

As illustrated in FIG. 3A, the connection pipe portion 472 can be exposed to the outside (to the air) by protruding outward from a second sidewall 40-2 of the cartridge 4 on the opposite side of the first sidewall 40-1 (side facing the first sidewall 40-1). In this case, some of heat absorbed by the cooling medium inside the cartridge 4 while flowing along the first pipe portion 470 is released to the air through the connection pipe portion 472 exposed to the outside, so that the cooling medium can recover its cooling performance.

According to an embodiment, as illustrated in FIG. 3B, heat dissipation fins 473 may also be attached to the connection pipe portion 472. The heat dissipation fins 473 may be attached to or formed on the surface of the connection pipe portion 472 protruding outward from the first sidewall 40-1 and exposed to the outside (air). In this case, the surface area of the connection pipe portion 472 coming into contact with the outside air is increased by the heat dissipation fins 473 attached to the surface of the connection pipe portion 472, so that the cooling performance of the cooling medium can be recovered more quickly.

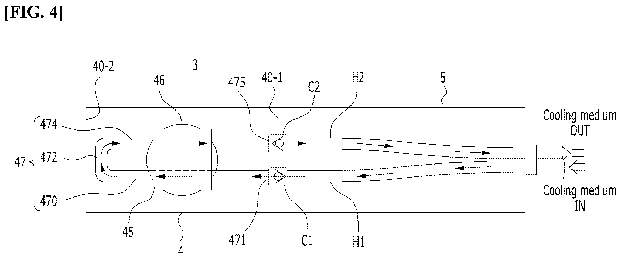

FIG. 4 is a planar schematic view of the present disclosure illustrating a preferred another embodiment of the shaft, and the connection pipe portion 472 may also have a structure that is completely embedded in the internal space (first space) of the cartridge 4. In this case, since the connection pipe portion 472 is not exposed to the outside, it is difficult to expect the same effect (effect of recovering the cooling performance of the cooling medium through heat exchange with the outside air) as in the previous embodiment (see FIG. 3), but the structure is advantageous in terms of miniaturization or compactness of the device.

The inlet 471 of the first pipe portion 470 and the outlet 475 of the second pipe portion 474 may be provided with a first connection port C1 and a second connection port C2, respectively. The inlet 471 can be connected to a supply pipe H1, which is introduced from the outside into the inside of the handpiece 5, through the first connection port C1 so that the cooling medium can flow, and can be connected to a discharge pipe H2, which is drawn out from the inside of the handpiece 5 to the outside, through the second connection port C2 so that the cooling medium can flow.

The first connection port C1 and the second connection port C2 may be tubular fluid couplers. The first connection port C1 and the second connection port C2 may each be, for example, a known quick coupler including a socket part and a plug part. In this case, when the cartridge 4 is coupled to a designated position of the handpiece 5, the socket part and the plug part separated from each other can be connected in a one-touch manner, so that the inlet 471 and the supply pipe H1 can be connected to each other and the outlet 475 and the discharge pipe H2 can be connected to each other.

The cooling medium that takes heat from the ultrasound transmission medium while flowing along the shaft 47 may be water or air. The cooling medium can be supplied to the first pipe portion 470 of the shaft 47 through the supply pipe H1 in a cooled state by a cooling module, such as a chiller, provided in the main body 2 (see FIG. 1) according to the operation of a pump (not illustrated), and can be recirculated back into the cooling module through the discharge pipe H2 via the connection pipe portion 472 and the second pipe portion 474.

When the cooling medium is water, a check valve (reference numeral is omitted), which prevents the backflow of the cooling medium, that is, allows cooling water to flow only in one predetermined direction, can be installed in the first connection port C1 and the second connection port C2. In this case, the check valve of the first connection port C1 can be arranged so that the cooling medium flows only in the direction from the supply pipe H1 to the first pipe portion 470, and the check valve of the second connection port C2 can be arranged so that the cooling medium flows only in the direction from the second pipe portion 474 to the discharge pipe H2.

On the other hand, the external rotating shaft 54 and the internal rotating shaft 44 can be physically coupled and rotated in synchronization with each other (see FIG. 2 above). Such coupling (that is, connection) can be made in a direct or indirect manner, and the embodiments of the present invention are not limited to such a coupling method, and the external rotating shaft 54 and the internal rotating shaft 44 can be connected in various manners.

For example, according to an embodiment, the external rotating shaft 54 and the internal rotating shaft 44 can be indirectly coupled to each other through a magnet coupler 6 with a first sidewall 40-1 of the cartridge 4 interposed therebetween and can be rotated in synchronization with each other. That is, the external rotating shaft 54 and the internal rotating shaft 44 can be magnetically coupled to each other through the magnet coupler 6 with the first sidewall 40-1 interposed therebetween.

The magnetic coupler 6 may include a pair of couplers 60a and 60b. The pair of couplers may be a coupler installed on the external rotating shaft 54 (hereinafter, referred to as a 'first coupler 60a') and a coupler installed on the internal rotating shaft 44 (hereinafter, referred to as a 'second coupler 60b'). The first coupler 60a can be configured at an end of the external rotating shaft 54 adjacent to the first sidewall 40-1, and the second coupler 60b can be configured at an end of the internal rotating shaft 44 adjacent to the first sidewall 40-1.

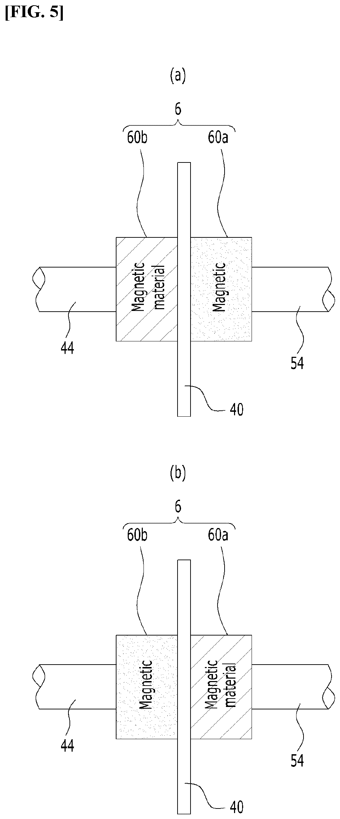

The first coupler 60a can be configured as an integral portion or coupled portion at the end of the external rotating shaft 54, thereby enabling a rotational motion integrated with the external rotating shaft 54. The second coupler 60b can be configured as an integral portion or coupled portion at the end of the internal rotating shaft 44 and magnetically coupled with the first coupler 60a with the first sidewall 40-1interposed therebetween, thereby enabling a rotational motion synchronized with the first coupler 60a. FIG. 5 is a view illustrating a preferred embodiment of the magnet coupler illustrated in FIG. 2.

As an embodiment, as illustrated in FIG. 5, one of the first coupler 60a and the second coupler 60b may be configured as a circular permanent magnet, and the other may be a circular magnetic material, for example, a steel plate. For example, when the first coupler 60a is a permanent magnet, the second coupler 60b may be a magnetic material (see FIG. 5A). In contrast, when the first coupler 60a is a magnetic material, the second coupler 60b may be a permanent magnet (see FIG. 5B).

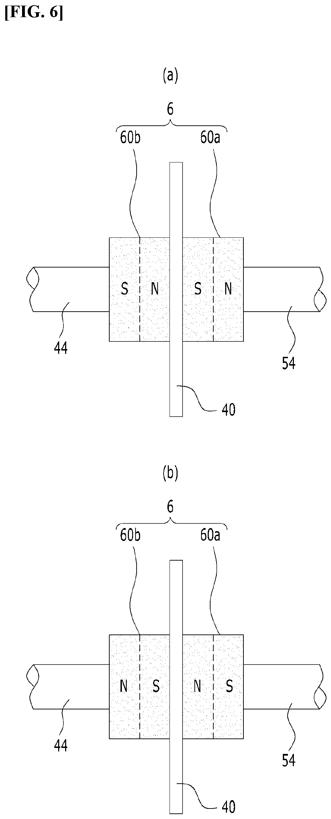

According to an embodiment, as illustrated in FIG. 6, for the magnetic coupling, both the first coupler 60a and the second coupler 60b can be configured as permanent magnets, preferably, circular permanent magnets. In this case, the magnetic poles of the surface of the first coupler 60a and the surface of the second coupler 60b, which are permanent magnets that are in close contact with each other with the first sidewall 40-1 interposed therebetween, may be opposite to each other so that a mutually attractive force acts.

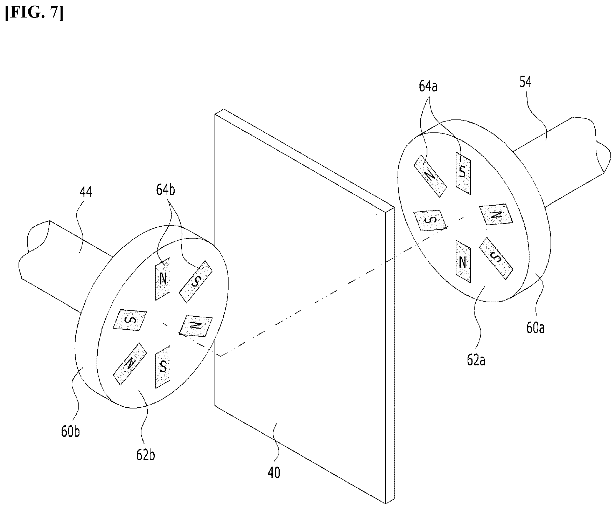

According to an embodiment, as illustrated in FIG. 7, the magnetic coupling can also be implemented through a configuration in which coupling magnets 64a and 64b are separately mounted on a surface 62a of the first coupler 60a and a surface 62b of the second coupler 60b, the surface 62a and the surface 62b facing each other with the first sidewall 40-1 interposed therebetween.

In this case, the coupling magnets 64a and 64b can be arranged at uniform intervals along the rotational direction on the surfaces 62a and 62b of the couplers 60a and 60b so that a relative slip in the rotational direction and a rotational force transmission delay due to the relative slip do not occur when a rotational force is transmitted from the first coupler 60a to the second coupler 60b.

The coupling magnets 64a mounted on the surface 62a of the first coupler 60a can be arranged so that the magnetic poles of sides exposed to the outside (sides in contact with the first sidewall) are alternated with respect to the rotation direction. The coupling magnets 64b mounted on the surface 62b of the second coupler 60b can also be arranged so that the magnetic poles of sides exposed to the outside (sides in contact with the first sidewall) are alternated with respect to the rotation direction.

The first coupler 60a and the second coupler 60b, which implement coupling between the shafts (the internal rotating shaft and the external rotating shaft), are indirectly connected through magnetic coupling with the first sidewall 40-1 interposed therebetween. Therefore, a portion of the first sidewall 40-1 that directly contacts each coupler may be worn during rotation. The wear is accelerated as the rotation speed increases, so that the life of the device may be shortened. Accordingly, it is necessary to reflect a method for suppressing or slowing down the wear in the configuration.

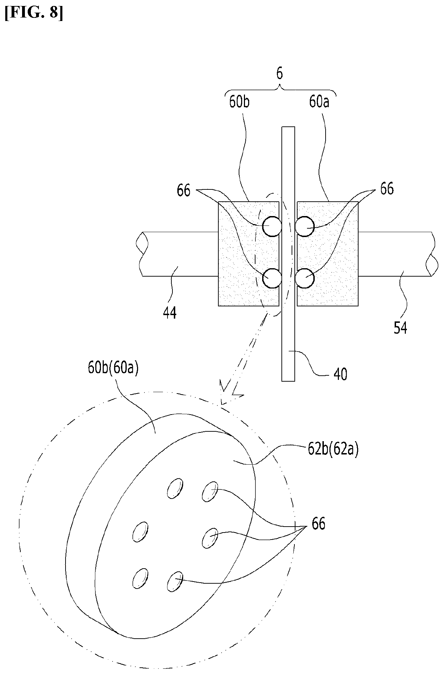

As an embodiment, as illustrated in FIG. 8, a plurality of balls 66 or needle pins may be installed on the surface of the first coupler 60a and the surface of the second coupler 60b facing each other with the first sidewall 40-1 interposed therebetween. At least a part of the balls 66 or the needle pins may protrude from the surfaces 62a and 62b facing each other. In this case, the protruding balls 66 or the needle pins support the rotational motion of the coupler while making point or line contact with the first sidewall 40 and performing a rolling motion, so that the wear of the first sidewall 40-1 can be significantly reduced.

As described above, the first coupler 60a and the second coupler 60b can be indirectly coupled by magnetic coupling with the first sidewall 40-1 interposed therebetween. Accordingly, the coaxiality between the two couplers may be distorted during the process of transmitting the rotational force, resulting in a loss of the rotational force. Therefore, a method for stably maintaining the coaxiality between the two couplers is preferably reflected in the configuration.

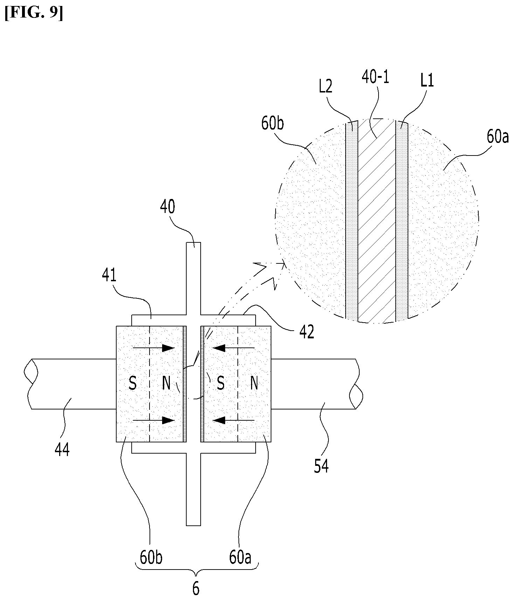

As an embodiment, as illustrated in FIG. 9, a ring-shaped internal rotation guide 41 can be formed on the inner surface of the first sidewall 40-1, and a ring-shaped external rotation guide 42 may be formed on the outer surface of the first sidewall 40-1 corresponding to the internal rotation guide 41. In this case, the second coupler 60b and the first coupler 60a can be respectively arranged in internal and external coupler receiving portions, which are respectively partitioned in the inside and the outside of the first sidewall 40-1 by the internal rotation guide 41 and the external rotation guide 42, thereby preventing coaxial misalignment.

In the embodiment of FIG. 9, a first lubricating layer L1 can be formed by a lubricant between the first coupler 60a and a first sidewall 40-1 partitioning the external coupler receiving portion. In addition, a second lubricating layer L2 can be formed by a lubricant between the second coupler 60b and the first sidewall 40-1 partitioning the inner coupler receiving portion. The lubricant is preferably a viscous semi-solid grease, but is not limited thereto.

According to such a configuration, direct contact between the pair of couplers 60a and 60b and the first sidewall 40-1 is blocked by the first lubricating layer L1 and the second lubricating layer L2, and thus the wear of the first sidewall 40-1 can be suppressed by the lubricating action of the lubricating layers. In addition, since the rotational load during the rotation of the couplers is reduced due to the lubricating action of the lubricating layers L1 and L2 and power consumption is reduced, thereby exhibiting an effect of improving the overall energy efficiency of the device.

However, as described above, the embodiments of the present invention are not limited to the coupling method of the external rotating shaft 54 and the internal rotating shaft 44 illustrated in FIGS. 2 and 5, and it should be understood that the coupling method described with reference to FIG. 2 is merely an example of various methods. That is, it should be understood that the structure of the shaft 47 serving as the heat exchange pipe described with reference to FIGS. 3 and 4 can be applied not only to the coupling method described with reference to FIGS. 2 and 5, but also to various coupling methods.

According to the embodiments of the present invention described above, the high intensive focused ultrasound probe is configured so that the cooling medium moves along the shaft that guides the linear motion of the transducer within the cartridge and cools the liquid ultrasound transmission medium (for example, degassed water) filled in the cartridge. Accordingly, an increase in the temperature of the cartridge can be suppressed, and problems of the related art, such as discomfort or burns caused by an increase in the temperature of the cartridge, can be solved.

In particular, since the shaft also serves as the heat exchange pipe (cooling pipe) for cooling the ultrasound transmission medium, no separate additional configuration is required to suppress an increase in the temperature of the cartridge. That is, it has the advantage of being an efficient configuration that can implement stable linear motion of the transducer and cartridge cooling with one shaft, and since no separate additional configuration is required for cooling implementation, a high-functionality product with an added cooling function can be provided at low cost.

The above description is merely intended to illustratively describe the technical spirit of the present disclosure, and various changes and modifications can be made by those skilled in the art to which the present disclosure pertains without departing from the essential features of the present disclosure.

Therefore, the embodiments disclosed in the present disclosure are not intended to limit the technical spirit of the present disclosure, but are intended to describe the present disclosure. The scope of the technical spirit of the present disclosure is not limited by these embodiments. The scope of the present disclosure should be interpreted by the accompanying claims and all technical spirits falling within the equivalent scope thereto should be interpreted as being included in the scope of the present disclosure.

## Claims

1. A high intensive focused ultrasound probe that emits high intensive focused ultrasound to skin, comprising:
a cartridge having an internal space filled with a liquid ultrasound transmission medium;
a handpiece to which the cartridge is coupled;
a transducer arranged in the internal space of the cartridge and configured to generate high intensive focused ultrasound from an input power source and output the generated high intensive focused ultrasound; and
a shaft configured to guide a one-dimensional linear motion of the transducer with respect to a specific direction in the internal space,
wherein the shaft is configured in a form of a hollow pipe having an empty interior, and
the shaft having the form of a hollow pipe serves as a heat exchange pipe that circulates a cooling medium in the internal space.

2. The high intensive focused ultrasound probe of claim 1, wherein the shaft comprises:
a first pipe portion into which the cooling medium is introduced through an inlet formed on a side of a first sidewall of the cartridge;
a second pipe portion parallel to the first pipe portion and through which the cooling medium is discharged through an outlet formed on the side of the first sidewall; and
a connection pipe portion configured to connect the first pipe portion and the second pipe portion so that the cooling medium is able to flow.

3. The high intensive focused ultrasound probe of claim 2, wherein at least a part of the connection pipe portion protrudes outward from a second sidewall of the cartridge on an opposite side of the first sidewall and is exposed to an outside.

4. The high intensive focused ultrasound probe of claim 3, wherein heat dissipation fins are attached to a surface of the connection pipe portion protruding outward from the first sidewall and exposed to the outside.

5. The high intensive focused ultrasound probe of claim 2, wherein the connection pipe portion is arranged in the internal space.

6. The high intensive focused ultrasound probe of claim 2, wherein a supply pipe introduced from an outside into an inside of the handpiece is connected to the inlet of the first pipe portion through a first connection port, and
a discharge pipe drawn out from the inside of the handpiece to the outside is connected to the outlet of the second pipe portion through a second connection port.

7. The high intensive focused ultrasound probe of claim 6, wherein a check valve is installed in the first connection port and the second connection port to allow the cooling medium to flow only in one direction,
the check valve of the first connection port is arranged so that the cooling medium flows only in a direction from the supply pipe to the first pipe portion, and
the check valve of the second connection port is arranged so that the cooling medium flows only in a direction from the second pipe portion to the discharge pipe.

8. The high intensive focused ultrasound probe of claim 1, wherein the transducer is connected to a movable block moving along the shaft.

9. The high intensive focused ultrasound probe of claim 1, further comprising:
an external rotating shaft configured to rotate inside the handpiece by a motor;
an internal rotating shaft configured to move the transducer while rotating inside the cartridge; and
a magnet coupler configured to magnetically couple the external rotating shaft and the internal rotating shaft with a first sidewall of the cartridge interposed between the external rotating shaft and the internal rotating shaft.

10. The high intensive focused ultrasound probe of claim 9, wherein the magnet coupler comprises:
a first coupler coupled to the external rotating shaft and making a synchronized rotational motion; and
a second coupler coupled to the internal rotating shaft and forming magnetic coupling with the first coupler with the first sidewall interposed between the first coupler and the second coupler.

11. The high intensive focused ultrasound probe of claim 9, wherein threads are formed on a peripheral surface of the internal rotating shaft, and
the movable block is formed with a fastening hole screw-coupled with the threads of the internal rotating shaft.

12. The high intensive focused ultrasound probe of claim 9, wherein an internal space of the cartridge is divided by a space partition plate into a first space and a second space isolated from the first space.

13. The high intensive focused ultrasound probe of claim 12, wherein the first space is filled with a liquid ultrasound transmission medium,
the transducer and the internal rotating shaft are arranged in the first space filled with the liquid ultrasound transmission medium, and
a circuit board that controls the transducer is arranged in the second space.
